(19) Europäisches Patentamt
European Patent Office
Office européen des brevets

(11) Numéro de publication : **0 284 521 B1**

# (12) FASCICULE DE BREVET EUROPEEN

(45) Date de publication du fascicule du brevet : **15.01.92 Bulletin 92/03**

(51) Int. Cl.⁵ : **C08L 83/04,** A61K 9/22, C08K 13/02, A61K 33/18, C02F 1/76

(21) Numéro de dépôt : **88420065.0**

(22) Date de dépôt : **23.02.88**

## (54) Composition élastomère silicone polyaddition contenant de l'iode.

(30) Priorité : **26.02.87 FR 8702884**

(43) Date de publication de la demande : **28.09.88 Bulletin 88/39**

(45) Mention de la délivrance du brevet : **15.01.92 Bulletin 92/03**

(84) Etats contractants désignés : **AT BE CH DE ES FR GB GR IT LI LU NL SE**

(56) Documents cités :
**DE-A- 1 467 861**
**GB-A- 1 412 970**
**US-A- 2 918 400**
**US-A- 4 275 194**
**US-A- 4 384 960**
**US-A- 4 420 590**
**US-A- 4 640 956**

(73) Titulaire : **RHONE-POULENC CHIMIE**
**25, quai Paul Doumer**
**F-92408 Courbevoie Cédex (FR)**

(72) Inventeur : **Cyprien, Guy**
**7, rue d'Anjou**
**F-94240 l'Hay Les Roses (FR)**
Inventeur : **Fisch, Alain**
**22, rue de l'Yvette**
**F-75016 Paris (FR)**
Inventeur : **Haggiage, Johnny**
**72, rue du Fort Saint-Irénée**
**F-69005 Lyon (FR)**
Inventeur : **Porte, Hugues**
**6, chemin de Crépieux**
**F-69300 Caluire (FR)**
Inventeur : **Prazuck, Thierry**
**53, avenue Mathurin Moreau**
**F-75019 Paris (FR)**
Inventeur : **Torres, Ghislaine**
**104, rue Ney**
**F-69006 Lyon (FR)**

(74) Mandataire : **Fabre, Madeleine-France et al**
**RHONE-POULENC CHIMIE Direction des Brevets Secteur Spécialités Chimiques 25, quai Paul-Doumer**
**F-92408 Courbevoie Cédex (FR)**

EP 0 284 521 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition élastomère de silicone polyaddition contenant de l'iode ainsi qu'un procédé de traitement des eaux d'usage domestique et de boissons à l'aide de cette composition.

On estime actuellement à plusieurs centaines de millions le nombre de sujets présentant une déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc.....).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'alors sont demeurées inefficaces.

Ces actions visent essentiellement d'une part :

— l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu'il n'est pas parfaitement emballé.

et d'autre part :

— l'injection intra-musculaire d'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induite par l'injection d'une dose nécessairement supra-physiologique.

On a par ailleurs décrit dans le brevet belge BE-A-889680 l'introduction d'oligo-éléments, dont l'iode, dans l'eau de boisson des ruminants sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A2347567, US-A-2743208 et US-A-3408295.

Il existe également de très nombreux brevets décrivant l'utilisation de systèmes polymères en particulier de silicone, pour la libération contrôlée d'un principe actif au moyen par exemple d'un système transdermique (brevet américain US-A-4053580), ou par absorption buccale notamment pour des ruminants (brevet français FR-A-2560768).

Enfin par le brevet américain US-A-4384960 il est décrit la mise en pastilles d'iode $I_2$ dans une bouteille en plastique dans laquelle l'eau pénètre par une membrane poreuse. L'eau solubilise l'iode. Le rôle de la membrane est seulement d'éviter que l'iode en pastilles ne sorte pas de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car $I_2$ est un inhibiteur bien connu des catalyseurs de durcissement des élastomères silicones vulcanisables à température ambiante comme indiqué en particulier dans la publication de W.D. MORAIN et al. Plastic and Reconstructive Surgery 59, 2, 215-222 (1977).

Dans cette publication il est indiqué que l'iode moléculaire $I_2$ est un inhibiteur de l'action catalytique du platine et empêche donc le durcissement des compositions silicones polyaddition.

En outre dans le système du brevet US-A 4384960 il n'y a pas de contrôle de la libération de l'iode d'une part, et, d'autre part, l'iodation de l'eau se fait par addition discontinue ou en continu de quelques gouttes d'eau très iodée (à saturation) dans la bouteille, dans un récipient quelconque contenant de l'eau non traitée. Il est clair que la solution proposée par US-A-4384960 est imparfaite par le fait notamment qu'il s'agit d'une technique individuelle qui, comme l'injection intra-musculaire d'iode, nécessite une éducation et une mobilisation massive des populations.

Le brevet US-A-4420590 enseigne que de l'eau peut être rendue potable après passage au contact d'une résine échangeuse d'anion traitée par essentiellement :

— de l'iode élémentaire,

— du bromure de potassium,

— de l'iodure de potassium, dans des proportions relatives bien définies.

Le brevet US-A-4384960 enseigne qu'il est possible de prévenir les conséquences d'une carence en iode en ajoutant de l'iode élémentaire (c'est-à-dire au degré d'oxydation 0) à de l'eau.

Le brevet anglais GB-A-1412970 enseigne que des substances pharmacologiquement actives, non ioniques et lipophiles, peuvent être libérées dans l'organisme après qu'elles aient été mélangées avec un élastomère silicone vulcanisant à froid, le produit résultant de ce mélange étant mis sous la forme filamentaire et introduit dans l'organisme ou mis à son contact.

La présente invention a précisément pour but de proposer une composition élastomère de silicone polyaddition contenant de l'iode utilisable pour le traitement continu des eaux d'usage domestique, en particulier dans les systèmes d'adduction, de traitement des eaux dans les puits et les forages et qui permette de relarguer (libérer) une quantité contrôlée et adaptée d'iode en vue d'assurer l'éradication des diverses maladies dues à une carence en iode.

Elle a également pour but de proposer une composition élastomère de silicone polyaddition contenant de l'iode qui immergée de façon appropriée dans les endroits contenant l'eau à traiter, en particulier les puits et forages, relargue (libère) de façon continue pendant au moins six mois, de préférence pendant au moins un an, une quantité appropriée d'iode sous une forme et à une dose thérapeutiquement active et efficace pour soigner les diverses maladies dues à une carence en iode.

Elle a également pour but de proposer une composition élastomère de silicone polyaddition contenant de l'iode qui, immergée de façon appropriée dans les endroits contenant l'eau à traiter n'ait aucune action secondaire indésirable et néfaste sur le plan chimique et biologique sur les eaux à traiter.

Elle a également pour but de proposer une composition élastomère de silicone polyaddition contenant de l'iode présentant une forme adaptée au milieu dans lequel se trouve l'eau à traiter, cette forme étant adaptée en particulier aux puits et/ou forages et présentant un système d'introduction dans les puits et/ou forages permettant son remplacement aisé.

Elle a également pour but de proposer une composition élastomère de silicone polyaddition contenant de l'iode apporté par un composé de l'iode qui n'inhibe pas le durcissement (réticulation) de la composition de silicone polyaddition en un élastomère.

Ces buts et d'autres sont atteints par la présente invention qui concerne en effet une composition de silicone polyaddition durcissable en un élastomère par des réactions d'hydrosilylation, caractérisée en ce qu'elle comporte :

— (A) : au moins un organopolysiloxane présentant par molécule au moins deux groupes vinyle liés au silicium, et ayant une viscosité inférieure à 500000 mPa · s,

— (B) : au moins un organopolysiloxane présentant par molécule au moins trois atomes d'hydrogène liés au silicium, le rapport molaire des atomes d'hydrogène liés au silicium dans (B) aux radicaux vinyle liés au silicium dans (A) étant compris entre 0,4 et 10,

— (C) : une quantité catalytiquement efficace d'un catalyseur qui est un composé d'un métal du groupe du platine,

— (D) : au moins un composé de l'iode à l'exception de l'iode moléculaire $I_2$, sous forme solide à température ambiante, soluble dans l'eau, non toxique, et n'inhibant pas le durcissement de ladite composition, dans laquelle ce composé de l'iode est dispersé de façon homogène.

Dans ce qui suit ou ce qui précède, sauf mentions contraires, les pourcentages et parties sont en poids.

La composition de silicone peut comporter de 5 à 130 parties, de préférence de 10 à 100 parties de composé d'iode (D) pour 100 parties de la somme (A) + (B).

Les quantités de (A) et (B) sont choisies de manière que le rapport molaire des atomes d'hydrogène liés au silicium dans (B) aux radicaux vinyle liés au silicium dans (A) soit compris généralement entre 0,4 et 10, de préférence entre 0,6 et 5.

Les groupes vinyle dans (A) et les atomes d'hydrogène dans (B) sont généralement liés à des atomes de silicium différents.

Comme composé de l'iode on peut utiliser :

— les iodures ou iodates de formules générales :

$(M^{a+})\ (I^-)_a$

et $(M^{a+})\ (IO_3^-)_a$

dans laquelle a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NT)^+$, dont les radicaux T identiques ou différents représentent un radical alkyle linéaire ou ramifié en $C_1$-$C_{20}$ ou un atome d'hydrogène, tel que l'ion ammonium $NH^+_4$

Les cations $M^{a+}$ et $NT_4^+$, sont choisis de telle sorte que l'iodure ou l'iodate correspondant soit un solide à température ambiante, soit soluble dans l'eau et soit non toxique.

Comme iodures et iodates on peut en particulier utiliser ceux de formules :

| | |
|---|---|
| Na I, | Na $IO_3$, |
| K I, | K $IO_3$, |
| Mg $I_2$, | Mg $I_2 \cdot 8H_2O$, |
| $Mg(IO_3)_2 \cdot 4H_2O$, | |
| $NH_4$ I | |
| Fe $I_2 \cdot 4H_2O$ | |
| Mn $I_2$ | |

Ces sels peuvent contenir de l'eau d'hydratation ou de l'eau de constitution.

Pour des raisons de facilité de mise en oeuvre les composés d'iode solides sont préférés, et parmi ceux-ci NaI et $KIO_3$ sont les plus préférés.

Tous les composés de l'iode tels que définis ci-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode (D) soluble dans l'eau on entend un composé présentant une solubilité d'au moins 100 µg/l à température ambiante.

Par ailleurs les composés de l'iode (D) ne doivent pas inhiber le durcissement de la composition de silicone en élastomère. L'iode moléculaire $I_2$ est donc exclu des composés de l'iode (D) utilisable dans le cadre de la présente invention.

En particulier dans les pays en développement, l'eau à usage domestique (boisson, lavage, irrigation, etc.....) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite à l'aide d'une pompe à corps immergé.

Cette eau se renouvelle principalement en fonction de l'utilisation du forage qui dépend de la saison. En effet en saison des pluies le forage est traditionnellement moins utilisé. Par contre en saison sèche, le forage débite environ 12 heures par jour, soit une quantité comprise entre 5 et 10 $m^3$ par jour pendant environ six mois.

En règle générale, un puits peut être asséché deux fois durant la journée au moment de la saison sèche ce qui correspond avec ces données statistiques moyennes, à un maximum d'utilisation de 5 à 10 $m^3$.

De nombreuses études montrent que dans les zones de grande endémie goîtreuse, le taux pré-existant d'équivalent en iode dans l'eau des forages ou des puits est inférieur à 2 microgrammes par litre (2 µg/l). Il est estimé actuellement qu'un apport journalier d'environ 100 µg d'équivalent iode par jour et par personne serait suffisant pour prévenir le développement d'un goître endémique et sans doute environ 150 µg lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 µg, de préférence environ 100 µg d'équivalent iode par jour constitue le but recherché.

Ainsi sachant qu'un individu adulte absorbe en moyenne 2 litres d'eau par jour et sur la base des données ci-dessus (forage ayant un débit de 600 1/h), il apparaît souhaitable qu'un litre d'eau traitée contienne environ 50 µg/l d'iode, ce qui correspond à 50 µg d'équivalent iode par litre et par personne, ce qui nécessite que la composition de silicone relargue 720 mg/j d'équivalent d'iode, soit 270 g d'équivalent iode à relarguer pendant une année.

Dans ce qui suit ou ce qui précède, sauf indications contraires, les parties et pourcentages sont en poids.

De façon surprenante et inattendue on a en effet découvert selon la présente invention qu'il est possible d'ajouter comme charge de bourrage dans une composition élastomère silicone polyaddition des quantités importantes de composé d'iode sous une forme solide tel que défini ci-dessus, à savoir p.ex. de 5 à 130 parties, de préférence de 10 à 100 pour 100 parties de la somme des organopolysiloxanes (A) + (B) éventuellement préalablement chargés avec une charge siliceuse renforçante ou semi-renforçante et d'obtenir ainsi un élastomère réticulé qui présente des caractéristiques mécaniques suffisantes pour l'application envisagée et qui,

immergé dans l'eau permette d'assurer un relargage continu et contrôlé d'iode, de préférence pendant au moins un an.

Le système de libération contrôlée de l'iode fait partie des systèmes matriciels dont la diffusion du principe actif est normalement régi par la loi de FICK, c'est-à-dire par une cinétique de diffusion d'ordre 1/2 pour seulement 60% en poids du principe actif. Au-delà de 60% il y a épuisement de la matrice et les flux de diffusion sont fortement diminués. De façon surprenante et inattendue, on a trouvé que le système matriciel silicone conforme à l'invention libère l'iode suivant une cinétique d'ordre 0 et de façon continue et cela jusqu'à ce que 80% en poids et plus du composé d'iode soit libéré.

L'avantage considérable apporté par la matrice silicone est donc qu'il est très facile d'extrapoler la diffusion continue du principe actif après une mesure de la quantité libérée au bout d'au moins un mois puisque l'on sait que la cinétique de diffusion est d'ordre 0 et qu'au moins 80% du composé d'iode sera libéré suivant cette cinétique.

De façon à maîtriser la libération du principe actif, il est avantageux de présenter la matrice silicone sous la forme de modules (éléments) élémentaires de formes variées tels que des cubes, des parallélépipèdes rectangles, des cylindres, des sphères dont les paramètres fondamentaux sont les suivants :

— la nature du composé d'iode,
— le diamètre moyen (granulométrie) g des particules du composé d'iode dans le cas préféré où ce dernier est un solide,
— la teneur t de composé d'iode au sein de la matrice,
— le rapport R de la surface au volume du module.

La nature du composé d'iode et sa granulométrie définissent la vitesse de diffusion V à travers la matrice, du principe actif.

Plus g est petit et plus V est lent et inversement.

Plus t est grand et plus le flux de principe actif est élevé et inversement.

Plus R est grand et plus le flux élué de principe actif est grand et inversement.

L'homme de métier par des expériences de routine peut, sans difficultés, aboutir rapidement au résultat visé en extrapolant le temps d'élution théorique qui correspondra au temps réel de diffusion du principe actif.

Pour NaI et $KIO_3$ qui sont les composés d'iode préférés, g, t et R peuvent être avantageusement choisis dans les zones suivantes :

— g compris entre 1 et 300 μm,
— t compris entre 10 et 100 parties en poids de composé d'iode pour 100 parties de (A),
— R compris entre 0,5 et 50 pour une forme cylindrique.

Le composé d'iode est dispersé de façon homogène au sein de la matrice.

Les diverses bases de composition élastomère de silicone polyaddition ne contenant pas le composé d'iode (D) sont bien connues et décrites dans de très nombreuses publications et brevets. Elles sont disponibles dans le commerce.

Ces compositions réticulent par réaction d'addition (denommée également réaction d'hydrosilylation), catalysée par un composé d'un métal du groupe du platine, d'un groupe vinyle de l'organopolysiloxane (A) sur une fonction hydrure de l'organopolysiloxane (B).

L'organopolysiloxane vinylé (A) peut être un organopolysiloxane présentant des motifs siloxyle de formule:

$$Y_a Z_b SiO_{\frac{(4-a-b)}{2}} \qquad (1)$$

dans laquelle Y est un groupe vinyle, Z est un groupe hydrocarboné monovalent n'ayant pas d'action défavorable sur l'activité du catalyseur. Z est généralement choisi parmi les groupes alkyles ayant de 1 à 8 atomes de carbone inclus tels que les groupes méthyle, éthyle, propyle et 3,3,3-trifluoropropyle et les groupes aryle tels que xylyle et tolyle et phényle, a est 1 ou 2, b est 0,1 ou 2 et a + b est compris entre 1 et 3, éventuellement tous les autres motifs étant des motifs de formule moyenne :

$$Z_c SiO_{\frac{4-c}{2}} \qquad (2)$$

dans laquelle Z a la même signification que ci-dessus et c a une valeur comprise entre 0 et 3.

L'organopolysiloxane (B) peut être un organohydrogénopolysiloxane comportant des motifs siloxyle de formule :

$$H_dW_eSiO_{\frac{4-d-e}{2}} \quad (3)$$

dans laquelle W est un groupe hydrocarboné monovalent n'ayant pas d'action défavorable sur l'activité du catalyseur et répondant à la même définition que Z, d est 1 ou 2, e est 0,1 ou 2, d + e a une valeur comprise entre 1 et 3, éventuellement tous les autres motifs étant des motifs de formule moyenne :

$$W_gSiO_{\frac{4-g}{2}} \quad (4)$$

dans laquelle W a la même signification que ci-dessus, g a une valeur comprise entre 0 et 3. Toutes les valeurs limites de a, b, c, d, et et g sont incluses.

L'organopolysiloxane (A) peut être uniquement formé de motifs de formule (1) ou peut contenir en outre des motifs de formule (2).

L'organopolysiloxane (A) peut présenter une structure linéaire ramifiée cyclique ou en réseau. Le degré de polymérisation est 2 ou plus et est généralement inférieur à 5000. Par ailleurs si l'organopolysiloxane (A) est linéaire, il présente une viscosité à 25°C inférieure à 500000 mPa · s.

Z est généralement choisi parmi les radicaux méthyle, éthyle et phényle, 60% molaire au moins des radicaux Z étant des radicaux méthyle.

Les organopolysiloxanes (A) et (B) sont bien connus et sont par exemple décrits dans les brevets US-A-3220972, US-A-3284406, US-A-3436366, US-A-3697473 et US-A-4340709.

Des exemples de motifs siloxyle de formule (1) sont le motif vinyldiméthylsiloxyl, le motif vinylphénylméthylsiloxyl, le motif vinylsiloxyl et le motif vinylméthysiloxyl.

Des exemples de motifs siloxyle de formule (2) sont les motifs $SiO_{4/2}$, diméthylsiloxane, méthylphénylsiloxane, diphénylsiloxane, méthylsiloxane et phénylsiloxane.

Des exemples d'organopolysiloxanes (A) sont les diméthylpolysiloxanes à extrémités diméthylvinylsiloxyle, les copolymères méthylvinyldiméthylpolysiloxanes à extrémités triméthylsiloxyle, les copolymères méthylvinyldiméthylpolysiloxanes à extrémités diméthylvinylsiloxyle, les méthylvinylpolysiloxanes cycliques.

L'organopolysiloxane (B) peut être uniquement formé de motifs de formule (3) ou comporte en plus des motifs de formule (4).

L'organopolysiloxane (B) peut présenter une structure linéaire ramifiée cyclique ou en réseau. Le degré de polymérisation est 2 ou plus et est généralement inférieur à 5000.

Le groupe W a la même signification que le groupe Z ci-dessus.

Des exemples de motifs de formule (3) sont :

$$H(CH_3)_2SiO_{1/2}, HCH_3SiO_{2/2}, H(C_6H_5)SiO_{2/2}.$$

Les exemples de motifs de formule (4) sont les mêmes que ceux donnés plus haut pour les motifs de formule (2).

Des exemples d'organopolysiloxanes (B) sont :

— les diméthylpolysiloxanes à extrémités hydrogénodiméthylsilyle, les copolymères diméthylhydrogénométhylpolysiloxanes à extrémités triméthylsiloxyle, les copolymères diméthylhydrogénométhylpolysiloxanes à extrémités hydrogénodiméthylsiloxyle, les hydrogénométhylpolysiloxanes à extrémités triméthylsiloxyle, les hydrogénométhylpolysiloxanes cycliques.

Le rapport du nombre d'atomes d'hydrogène liés au silicium dans l'organopolysiloxane (B) sur le nombre de groupes à insaturation alcényle de l'organopolysiloxane (A) est compris entre 0,4 et 10, de préférence entre 0,6 et 5. Ce rapport peut être toutefois compris entre 2 et 5, si on souhaite faire des mousses d'élastomères.

L'organopolysiloxane (A) et/ou l'organopolysiloxane (2) peut être dilué dans un solvant organique non toxique compatible avec les silicones.

Les organopolysiloxanes (A) et (B) en réseau sont dénommés couramment résines silicones.

Les bases de compositions silicones polyaddition peuvent ne comporter que des organopolysiloxanes (1)

et (2) linéaires comme par exemple celles décrites dans les brevets américains précités : US-A-3220972, US-A-3697473 et US-A-4340709, ou comporter à la fois des organopolysiloxanes (A) et (B) ramifiés ou en réseau comme par exemple celles décrites dans les brevets américains précités ; US-A-3284406 et US-A 3436366.

Les catalyseurs (C) sont également bien connus.

On utilise de préférence les composés du platine et du rhodium.

On peut en particulier utiliser les complexes du platine et d'un produit organique décrit dans les brevets américains US-A-3159601, US-A-3159602, US-A-3220972 et les brevets européens EP-A-57459, EP-A-188978 et EP-A-190530, les complexes du platine et d'organosiloxane vinylé décrits dans les brevets américains : US-A-3 419 593, US-A-3715334, US-A-3377432 et US-A-3814730.

On peut en particulier utiliser les complexes du rhodium décrits dans les brevets britanniques : GB-A-1421136 et GB-A-1419769.

Le catalyseur généralement préféré est le platine.

Dans ce cas la quantité pondérale de catalyseur (C) calculée en poids de platine-métal est généralement comprise entre 2 et 600 ppm, en général entre 5 et 200 ppm basés sur le poids total des organopolysiloxanes (A) et (B).

Les compositions préférées, dans le cadre de la présente invention sont celles qui comportent :

- (A) : 100 parties d'une huile diorganopolysiloxane bloquée à chaque extrémité de sa chaîne par un motif vinyldiorganosiloxyle dont les radicaux organiques liés aux atomes de silicium sont choisis parmi les radicaux méthyle, éthyle et phényle, au moins 60% molaire de ces radicaux étant des radicaux méthyle de viscosité 100 à 500000, de préférence de 1000 à 200000 mPa · s à 25°C,
- (B) : au moins un organohydrogénopolysiloxane choisi parmi les homopolymères et les copolymères liquides linéaires ou en réseau présentant par molécule au moins 3 atomes d'hydrogène liés à des atomes de silicium différents et dont les radicaux organiques liés aux atomes de silicium sont choisis parmi les radicaux méthyle, éthyle et 60% au moins de ces radicaux étant des radicaux méthyle, le produit (B) étant utilisé en quantité telle que le rapport molaire des fonctions hydrure sur les groupes vinyle sont compris entre 1,1 et 4,
- (C) : une quantité catalytiquement efficace d'un catalyseur au platine,
- (D) : un composé de l'iode tel que défini ci-dessus.

De façon encore plus préférée jusqu'à 50% en poids du polymère (A) est remplacé par un copolymère en réseau comportant les motifs triméthylsiloxyle, méthylvinylsiloxyle et $SiO_{4/2}$ dans lequel 2,5 à 10% molaire des atomes de silicium comportent un groupe vinyle et dans lequel le rapport molaire des groupes triméthylsiloxyle au groupe $SiO_{4/2}$ est compris entre 0,5 et 1.

Les compositions selon l'invention peuvent comporter en outre des charges (E) renforçantes ou semi-renforçantes ou de bourrage, qui sont de préférence choisies parmi les charges siliceuses.

Les charges renforçantes sont choisies parmi les silices de combustion et les silices de précipitation. Elles ont une surface spécifique, mesurée selon les méthodes BET, d'au moins 50 $m^2/g$, de préférence supérieure à 70 $m^2/g$, une dimension moyenne des particules primaires inférieure à 0,1 micromètre ($\mu m$) et une densité apparente inférieure à 200 g/litre.

Ces silices peuvent être incorporées telles quelles ou de préférence après avoir été traitées par des composés organosiliciques habituellement utilisés pour cet usage. Parmi ces composés, figurent les méthylpolysiloxanes tels que l'hexaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthyldichlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20%, de préférence 18% environ.

Les charges semi-renforçantes ou de bourrage ont un diamètre particulaire supérieur à 0,1 $\mu m$ et sont choisies de préférence parmi le quartz broyé, les argiles calcinées et les terres de diatomées.

On peut généralement utiliser de 5 à 100 parties, de préférence de 5 à 50 parties de charge (E) pour 100 parties de la somme des organopolysiloxanes (A) + (B).

Les compositions polyaddition sont généralement stockées en deux emballages. En effet elles réticulent dès qu'on mélange tous ces constituants. Si l'on veut retarder cette réticulation pour obtenir une bonne homogénéisation du composé d'iode on peut ajouter à la composition un inhibiteur du catalyseur au platine. Ces inhibiteurs sont bien connus. On peut en particulier utiliser les amines organiques, les silazanes, les oximes organiques, les diesters de diacides carboxyliques, les alcools acétyléniques, les cétones acétyléniques, les vinylméthylcyclopolysiloxanes (voir par exemple US-A-3445420 et US-A-3989667). L'inhibiteur est utilisé à raison de 0,005 à 5 parties, de préférence 0,01 à 3 parties pour 100 parties du constituant (A).

Pour obtenir une bonne homogénéisation du composé d'iode, il est en effet souhaitable que la matrice silicone présente une certaine viscosité de l'ordre de 5000 à 30000 mPa · s à 25°C. Une telle viscosité peut être obtenue par une préréticulation, cette préréticulation étant bloquée à la viscosité désirée par addition d'un inhibiteur. On dispose ainsi de suffisamment de temps pour bien homogénéiser le composé d'iode au sein de la matrice silicone. La réticulation est alors achevée par chauffage de la matrice à une température telle que l'inhibiteur n'a plus d'effet sur l'action catalytique du platine.

Les compositions selon l'invention peuvent être malaxées à froid telles quelles et être extrudées ou moulées sous forme de modules (éléments) unitaires ; on peut par exemple mouler la composition sous la forme d'un cylindre de diamètre compris entre 0,5 et 9 cm. Après durcissement les cylindres de composition de silicone obtenues peuvent être découpées dans le cas de leur utilisation dans les forages à la longeur désirée de manière à ce que le cylindre comporte une quantité suffisante en équivalent iode pour un relargage de préférence pendant au moins une année. Au bout de cette période les cylindres sont remplacés.

De façon surprenante on a découvert que ces compositions de silicone réticulées ont des caractéristiques physiques suffisantes pour les applications envisagées et relarguent l'iode de façon continue et contrôlée de préférence pendant au moins un an.

Les exemples ci-après illustrent l'invention sans en limiter sa portée. Référence sera faite au dessin annexé sur lequel les figures 1 à 4 donnent la quantité libérée d'iode par rapport à la quantité initiale en fonction du temps.

Exemple 1.

* Préparation de la composante A.

On homogénéise à température ambiante dans un malaxeur, les constituants suivants :

a) 25 parties de résine silicone comportant 40% molaire de motifs $(CH_3)_3$ $SiO_{1/2}$, 6% molaire de motifs $(CH_3)(CH_2 = CH)SiO_{2/2}$ et 53,5% de motif $SiO_{4/2}$.

b) 75 parties d'une huile diméthylpolysiloxane bloquée à chacune des extrémités de sa chaine par un motif $(CH_3)$ $(CH_2 = CH)SiO_{1/2}$ de viscosité 3500 mPa · s à 25°C.

c) 40 ppm calculés en poids, de platine métal apporté par une solution à 0,25% d'acide hexachloroplatinique préparée par agitation à température ambiante de 0,6 partie d'acide hexachloro platinique, 10 parties d'isopropanol, 55 parties de xylène et 6 parties de tétraméthyl-1,1,3,3,divinyl-1,3 disiloxane.

* Préparation de la composante B.

On homogénéise à température ambiante, dans un malaxeur, les constituants suivants :

d) 45 parties de résine silicone liquide hydrogénée, préparée par hydrolyse de silicate d'éthyle et de $(CH_3)_2$ HSiCl en des quantités correspondant à une mole de SiO pour deux moles de $(CH_3)_2$ HSiCl en solution dans le toluène. Cette résine présente donc un rapport molaire théorique de motifs $(CH_3)HSiO_{1/2}$ de 2 et un rapport molaire réel de 2,23.

e) 12,5 parties de la résine a) de la partie (A).

f) 37,5 parties de l'huile vinylée b) de la partie (A).

La composition élastomère est obtenue par mélange de 10 parties de la composante A à 1 partie de la composante B.

* Préparation de la composition de l'exemple 1.

On incorpore à 100 parties de la composition élastomère 25 parties de NaI de granulométrie moyenne égale à 5 μm. Le mélange est agité, sous vide, pendant 22 minutes à 50°C. Lorsque la viscosité du mélange atteint 15000 mPa · s, l'agitation est stoppée et le mélange coulé dans un moule de diamètre 23 mm. La composition est ensuite durcie par chauffage à 100°C.

* Protocole expérimental de la mesure de la cinétique d'élution.

La composition élastomère contenant le NaI est découpé à longueur désirée (50 mm), conformément au rapport Surface/Volume (2,14 $cm^{-1}$) que l'on désire atteindre et immergée dans un récipient 600 ml d'eau distilée, thermostatée à 20°C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de

l'eau.

Des prélévements journaliers de 1ml sont réalisés dans les premiers temps de l'élution, et hebdomadaires au bout de 15 jours d'élution.

La concentration en iodure ou iodate, libérée par jour, est déterminée par un dosage avec une électrode spécifique à iodure :

A un millilitre de prélévement du récipient on rajoute deux millilitres d'une solution (K2SO4 + acide ascorbique) — cette solution joue le rôle de tampon ionique, et de solution réductrice dans le cas du dosage des iodates — et un millilitre d'eau distillée. On immerge l'électrode dans cette solution et on lit le potentiel électrochimique de la solution. Une courbe d'étalonnage préalablement établie avec des solutions en iodure de 5.10-5 M/l (M : mole) à 5.10-2 M/l permet de calculer la concentration (C) en iodure ou iodate en mg/l de la solution.

Les caractéristiques du cylindre immergé sont :

- Diamètre = 23 mm
- Hauteur = 50 mm
- Surface = 44,4 $cm^2$
- Volume = 20,76 $cm^3$
- S/V = 2,14 $cm^{-1}$
- Masse totale = 25,52 g
- Quantité initiale de I (Qo) = 4,27 g

Dans le tableau suivant (Tableau 1) sont rassemblés les résultats de la cinétique d'élution.

Q cumulé correspond à la quantité d'équivalent I (que nous appelons ion actif) éluée à l'instant t. Sachant que 80% en moles de l'ion actif incorporé élue selon une cinétique d'ordre zéro en fonction du temps, nous pouvons calculer pour chaque exemple le temps d'élution théorique (Te) selon :

$$Te = \frac{0,8 \times Qo}{\text{Flux journalier.}} \quad \textit{(jours)}$$

La courbe Q/Qo = f(t), ainsi que le Te de l'exemple, sont reportés sur la figure 1.

Exemple 2.

A 100 parties de la composition élastomère préparée conformément au mode opératoire de l'exemple 1 on ajoute 25 parties de NaI de granulométrie comprise entre 100 et 200 µm.

La préparation de l'échantillon est réalisée conformément au protocole décrit dans l'exemple 1.

On immerge dans 500 ml d'eau distillée, thermostatée à 20°C, un cylindre dont les caractéristiques sont les suivantes :

TABLEAU 1

| TEMPS (JOUR) | Q cumulé (gramme) ion acti | 100*Q/Q0 % |
|---|---|---|
| 0.12 | 0.013 | 0.29 |
| 0.29 | 0.024 | 0.58 |
| 1.00 | 0.048 | 1.14 |
| 2.00 | 0.063 | 1.48 |
| 6.00 | 0.101 | 2.37 |
| 8.00 | 0.126 | 2.96 |
| 12.00 | 0.158 | 3.72 |
| 15.00 | 0.183 | 4.31 |
| 21.00 | 0.250 | 5.89 |
| 26.00 | 0.270 | 6.35 |
| 33.00 | 0.398 | 9.38 |
| 40.00 | 0.440 | 10.36 |
| 49.00 | 0.542 | 12.76 |
| 61.00 | 0.545 | 12.85 |
| 79.00 | 0.845 | 19.91 |
| 96.00 | 1.098 | 25.87 |
| 117.00 | 1.195 | 28.14 |

* Diamètre : 23 mm
* Hauteur : 50 mm
* Surface : 44,4 $cm^2$
* Volume : 20,76 $cm^3$
* S/V : 2,14 $cm^{-1}$
* Masse totale : 25,03 g
* Quantité initiale de I (Qo) = 4,23 g

Le tableau 2 rassemble les résultats de la cinétique d'élution.
La courbe Q/Qo = f(t), ainsi que le Te de l'exemple sont reportés sur la figure 1 en annexe.

Exemple 3.

A 100 parties de la composition élastomère préparée conformément au mode opératoire défini à l'exemple

1, on incorpore 25 parties de NaI de granulométrie comprise entre 200 et 400 µm.

La préparation de l'échantillon est réalisée conformément au protocole décrit à l'exemple 1.

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$
* Masse totale : 25,52 g
* Quantité initiale de I (Qo) = 4,32 g

Le tableau 3 rassemble les résultats de la cinétique d'élution. La courbe Q/Qo = f(t), ainsi que le Te de l'exemple sont reportés sur la figure 1 en annexe.

TABLEAU 2

| TEMPS (JOUR) | Q cumulé (gramme) ion acti | 100*Q/Q0 % |
|---|---|---|
| 0.12 | 0.020 | 0.47 |
| 0.29 | 0.034 | 0.80 |
| 1.00 | 0.082 | 1.93 |
| 2.00 | 0.106 | 2.49 |
| 6.00 | 0.182 | 4.27 |
| 8.00 | 0.229 | 5.38 |
| 12.00 | 0.284 | 6.67 |
| 15.00 | 0.351 | 8.25 |
| 21.00 | 0.462 | 10.88 |
| 26.00 | 0.539 | 12.68 |
| 33.00 | 0.672 | 15.82 |
| 40.00 | 0.793 | 18.66 |
| 49.00 | 0.941 | 22.15 |
| 61.00 | 1.099 | 25.86 |
| 79.00 | 1.389 | 32.69 |
| 96.00 | 1.627 | 38.30 |
| 117.00 | 1.941 | 45.68 |

TABLEAU 3

| TEMPS (JOUR) | Q cumulé (gramme) ion acti | 100*Q/Q0 % |
|---|---|---|
| 0.12 | 0.029 | 0.66 |
| 0.29 | 0.047 | 1.09 |
| 1.00 | 0.109 | 2.51 |
| 2.00 | 0.162 | 3.74 |
| 6.00 | 0.290 | 6.70 |
| 8.00 | 0.352 | 8.14 |
| 12.00 | 0.477 | 11.03 |
| 15.00 | 0.563 | 13.04 |
| 21.00 | 0.738 | 17.07 |
| 26.00 | 0.823 | 19.04 |
| 33.00 | 1.220 | 28.22 |
| 40.00 | 1.465 | 33.89 |
| 49.00 | 1.537 | 35.56 |
| 61.00 | 1.583 | 36.63 |
| 79.00 | 2.250 | 52.06 |
| 96.00 | 2.894 | 66.99 |
| 117.00 | 3.004 | 69.52 |

Exemple 4.

A 100 parties de la composition élastomère préparée conformément au mode opératoire décrit à l'exemple 1, on incorpore 25 parties de NaI de granulométrie 100-200 µm.

La préparation de l'échantillon est réalisée conformément au protocole décrit à l'exemple 1.

On immerge dans 1000 ml d'eau distillée, thermostatée à 20°C, un cylindre dont les caractéristiques sont les suivantes :

* Diamètre : 36 mm
* Hauteur : 50 mm
* Surface : 76,9 $cm^2$
* Volume : 50,9 $cm^3$
* S/V : 1,51 $cm^{-1}$
* Masse totale : 60,77 g

* Quantité initiale de I (Qo) : 10,27 g

Le tableau 4 rassemble les résultats de la cinétique d'élution. La courbe Q/Qo = f(t), ainsi que le Te de l'exemple, sont reportés sur la figure 2 en annexe.

Exemple 5.

A 100 parties de la composition élastomère préparée conformément au mode opératoire défini à l'exemple 1, on incorpore 25 parties de NaI de granulométrie 100-200 µm.

La préparation de l'échantillon est réalisée conformément au protocole décrit à l'exemple 1.

On immerge dans 500 ml d'eau distillée, thermostatée à 20°C, un cylindre dont les caractéristiques sont les suivantes :

* Diamètre : 16 mm
* Hauteur : 50 mm
* Surface : 29,1 $cm^2$
* Volume : 10,03 $cm^3$
* S/V : 2,9 $cm^{-1}$
* Masse totale : 11,4 g
* Quantité initiale de I (Qo) : 1,93 g.

Le tableau 5 rassemble les résultats de la cinétique d'élution. La courbe Q/Qo = f(t), ainsi que le Te de l'exemple, sont reportés sur la figure 2 en annexe.

Exemple 6.

A 100 parties de la composition élastomère préparée conformément au mode opératoire défini à l'exemple 1, on incorpore 25 parties de NaI de granulométrie comprise entre 100 et 200 µm.

La préparation de l'échantillon est réalisée conformément au protocole opératoire décrit à l'exemple 1.

On immerge dans 500 ml d'eau distillée, thermostatée à 20°C, un cylindre dont les caractéristiques sont les suivantes :

* Diamètre : 11,7 mm
* Hauteur : 50 mm
* Surface : 20,5 $cm^2$
* Volume : 5,36 $cm^3$
* S/V : 3,82 $cm^{-1}$
* Masse totale : 6,4 g
* Quantité initiale de I (Qo) : 1,08 g

Le tableau 6 rassemble les résultats de la cinétique d'élution. La courbe Q/Qo = f(t), ainsi que le Te, sont reportés sur la figure 2 en annexe.

Exemple 7.

A 100 parties de la composition élastomère préparée conformément au mode opératoire défini à l'exemple 1, on incorpore 25 parties de NaI de granulométrie comprise entre 100 et 200 µm.

TABLEAU 4

| TEMPS (JOUR) | Q cumulé (gramme) ion acti | 100*Q/Q0 % |
|---|---|---|
| 0.35 | 0.102 | 0.99 |
| 1.04 | 0.147 | 1.43 |
| 4.10 | 0.245 | 2.38 |
| 6.08 | 0.321 | 3.12 |
| 8.01 | 0.429 | 4.17 |
| 11.06 | 0.549 | 5.34 |
| 22.27 | 0.792 | 7.70 |
| 28.36 | 0.855 | 8.31 |
| 39.20 | 1.462 | 14.21 |
| 49.09 | 1.765 | 17.15 |

TABLEAU 5

| TEMPS (JOUR) | Q cumulé (gramme) ion acti | 100*Q/Q0 % |
|---|---|---|
| 0.35 | 0.029 | 1.51 |
| 1.04 | 0.043 | 2.24 |
| 4.11 | 0.078 | 4.03 |
| 6.08 | 0.107 | 5.52 |
| 8.01 | 0.142 | 7.37 |
| 11.06 | 0.198 | 10.25 |
| 22.28 | 0.460 | 23.85 |
| 28.36 | 0.390 | 20.18 |
| 39.21 | 0.608 | 31.47 |
| 49.10 | 0.800 | 41.42 |

La préparation de l'échantillon est réalisée conformément au protocole décrit à l'exemple 1.

On immerge dans 400 ml d'eau distillée, thermostatée à 20°C, un cylindre dont les caractéristiques sont les suivantes :

* Diamètre : 9,7 mm
* Hauteur : 50 mm
* Surface : 16,7 $cm^2$
* Volume : 3,69 $cm^3$
* S/V : 4,52 $cm^{-1}$
* Masse totale : 4,52 g
* Quantité totale de I (Qo) = 0,76 g

Le tableau 7 rassemble les résultats de la cinétique d'élution. La courbe Q/Qo = f(t), ainsi que le Te, sont reportés sur la figure 2 en annexe.

Exemple 8.

A 100 parties de la composition élastomère préparée conformément au mode opératoire défini à l'exemple 1, on incorpore 16,5 parties de NaI de granulométrie 100-200 μm.

La préparation de l'échantillon est réalisée conformément au protocole opératoire défini dans l'exemple 1.

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$.
* Masse totale : 23,8 g
* Quantité initiale de I (Qo) = 2,85 g

Le tableau 8 rassemble les résultats de la cinétique d'élution. La courbe Q/Qo = f(t),ainsi que le Te, sont reportés sur la figure 3 en annexe.

TABLEAU 6

| TEMPS (JOUR) | Q cumulé (gramme) ion acti | 100*Q/Q0 % |
|---|---|---|
| 0.35 | 0.020 | 1.89 |
| 1.04 | 0.031 | 2.88 |
| 4.11 | 0.054 | 4.96 |
| 6.08 | 0.074 | 6.83 |
| 8.01 | 0.107 | 9.90 |
| 11.07 | 0.150 | 13.83 |
| 22.28 | 0.319 | 29.41 |
| 28.36 | 0.405 | 37.35 |
| 39.21 | 0.661 | 60.94 |
| 49.10 | 0.768 | 70.83 |

TABLEAU 7

| TEMPS (JOUR) | Q cumulé (gramme) ion acti | 100*Q/Q0 % |
|---|---|---|
| 0.35 | 0.017 | 2.22 |
| 1.04 | 0.027 | 3.54 |
| 4.11 | 0.053 | 6.86 |
| 6.08 | 0.075 | 9.83 |
| 8.02 | 0.109 | 14.23 |
| 11.07 | 0.164 | 21.42 |
| 22.29 | 0.351 | 45.92 |
| 28.37 | 0.440 | 57.50 |
| 39.21 | 0.647 | 84.59 |
| 49.10 | 0.686 | 89.68 |

TABLEAU 8

| TEMPS (JOUR) | Q cumulé (gramme) ion acti | 100*Q/Q0 % |
|---|---|---|
| 0.25 | 0.034 | 1.21 |
| 0.98 | 0.057 | 1.99 |
| 2.91 | 0.088 | 3.08 |
| 3.99 | 0.099 | 3.48 |
| 8.25 | 0.165 | 5.77 |
| 9.97 | 0.174 | 6.09 |
| 13.95 | 0.225 | 7.86 |
| 17.24 | 0.199 | 6.95 |
| 20.95 | 0.249 | 8.71 |
| 22.96 | 0.263 | 9.20 |
| 27.93 | 0.338 | 11.83 |
| 39.14 | 0.451 | 15.79 |
| 45.25 | 0.502 | 17.57 |
| 53.22 | 0.540 | 18.89 |
| 65.96 | 0.752 | 26.34 |

Exemple 9.

A 100 parties de la composition élastomère préparée conformément au mode opératoire défini à l'exemple 1, on incorpore 19 parties de NaI de granulométrie 100 à 200 µm.

La préparation de l'échantillon est réalisée conformément au protocole défini à l'exemple 1.

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$
* Masse totale : 24,34 g
* Quantité initiale de I (Qo) = 4,12 g

Le tableau 9 rassemble les résultats de la cinétique d'élution. La courbe Q/Qo = f(t), ainsi que le Te, sont reportés sur la figure 3 en annexe.

Exemple 10.

A 100 parties de la composition élastomère préparée conformément au mode opératoire défini à l'exemple 1, on incorpore 22 parties de NaI de granulométrie 100 à 200 μm.

La préparation de l'échantillon est réalisée conformément au protocole décrit à l'exemple 1.

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$
* Masse totale : 24,35 g
* Quantité initiale de I (Qo) = 3,71 g

TABLEAU 9

| TEMPS (JOUR) | Q cumulé (gramme) ion acti | 100*Q/Q0 % |
|---|---|---|
| 0.25 | 0.039 | 1.17 |
| 0.98 | 0.067 | 2.02 |
| 2.91 | 0.092 | 2.78 |
| 4.00 | 0.127 | 3.84 |
| 8.26 | 0.201 | 6.09 |
| 9.97 | 0.221 | 6.70 |
| 13.96 | 0.285 | 8.64 |
| 17.25 | 0.320 | 9.69 |
| 20.96 | 0.318 | 9.65 |
| 22.97 | 0.349 | 10.57 |
| 27.95 | 0.428 | 12.99 |
| 39.15 | 0.600 | 18.19 |
| 45.26 | 0.610 | 18.50 |
| 53.23 | 0.693 | 21.01 |
| 65.97 | 0.956 | 28.99 |

Le tableau 10 rassemble les résultats de la cinétique d'élution. La courbe Q/Qo = f(t) est reportée sur la

figure 3 en annexe.

Exemple 11.

A 100 parties de la composition élastomère prépararée conformément au mode opératoire défini à l'exemple 1, on incorpore 33,3 parties de NaI de granulométrie 100-200 μm.

La préparation de l'échantillon est réalisée conformément au protocole décrit à l'exemple 1.

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$
* Masse totale : 25,4 g
* Quantité initiale de I (Qo) : 5,37 g

Le tableau 11 rassemble les résultats de la cinétique d'élution. La courbe Q/Qo = f(T) ainsi que le Te, sont reportés sur la figure 3 en annexe.

Exemple 12.

A 100 parties de la composition élastomère préparée conformément au mode opératoire défini à l'exemple 1, on incorpore 25 parties de KIO3 de granulométrie moyenne égale à 5 μm. Le mélange est agité, sous vide, pendant 25 minutes à 50°C. Lorsque la viscosité du mélange atteint 15 000 mPa · s, l'agitation est stoppée et le mélange coulé dans un moule de diamètre 23 mm.
La composition est ensuite durcie par chauffage à 100°C pendant une heure.

TABLEAU 10

| TEMPS (JOUR) | Q cumulé (gramme) ion acti | 100*Q/Q0 % |
|---|---|---|
| 0.25 | 0.047 | 1.26 |
| 0.98 | 0.078 | 2.10 |
| 2.91 | 0.126 | 3.38 |
| 4.00 | 0.149 | 3.99 |
| 8.26 | 0.235 | 6.31 |
| 9.97 | 0.269 | 7.23 |
| 13.96 | 0.348 | 9.34 |
| 17.24 | 0.375 | 10.07 |
| 20.96 | 0.391 | 10.49 |
| 22.97 | 0.410 | 11.01 |
| 27.94 | 0.522 | 14.03 |
| 39.15 | 0.706 | 18.98 |
| 45.26 | 0.742 | 19.94 |
| 53.22 | 0.819 | 21.99 |
| 65.97 | 1.078 | 28.95 |

TABLEAU 11

| TEMPS (JOUR) | Q cumulé (gramme) ion acti | 100*Q/Q0 % |
|---|---|---|
| 0.25 | 0.069 | 1.29 |
| 0.99 | 0.122 | 2.27 |
| 2.91 | 0.210 | 3.91 |
| 4.00 | 0.250 | 4.65 |
| 8.26 | 0.440 | 8.19 |
| 9.97 | 0.488 | 9.08 |
| 13.97 | 0.656 | 12.20 |
| 17.25 | 0.707 | 13.15 |
| 20.97 | 0.722 | 13.43 |
| 22.97 | 0.782 | 14.54 |
| 27.95 | 0.947 | 17.60 |
| 39.16 | 1.200 | 22.32 |
| 45.26 | 1.187 | 22.08 |
| 53.23 | 1.350 | 25.10 |
| 65.97 | 1.811 | 33.67 |

On immerge dans 500 ml d'eau distillée, thermostatée à 20°C, un cylindre dont les caractéristiques sont les suivantes :

* Diamètre : 23 mm
* Hauteur : 50 mm
* Surface : 44,4 $cm^2$
* Volume : 20,76 $cm^3$
* S/V : 2,14 $cm^{-1}$
* Masse totale : 25 g
* Quantité initiale de I (Qo) : 2,96 g

Le tableau 12 rassemble les résultats de la cinétique d'élution. La courbe Q/Qo = f(t), ainsi que le Te, sont reportés sur la figure 4.

Exemple 13.

A 100 parties de la composition élastomère préparée conformément au mode opératoire décrit dans l'exemple 1, on incorpore 25 parties de KIO3 de granulométrie 100-200 µm.

La préparation de l'échantillon est réalisée conformément au protocole décrit à l'exemple 12.

On immerge dans 500 ml d'eau distillée, thermostatée à 20°C, un cylindre dont les caractéristiques sont identiques à celui de l'exemple 12.

Le tableau 13 rassemble les résultats de la cinétique d'élution. La courbe Q/Qo = f(t), ainsi que le Te, sont reportés sur la figure 4 en annexe.

Exemple 14.

A 100 parties de la composition élastomère préparée conformément au mode opératoire décrit à l'exemple 1, on incorpore 25 parties de KIO3 de granulométrie 200-400 μm.

La préparation de l'échantillon est réalisée conformément au protocole décrit dans l'exemple 13.

TABLEAU 12

| TEMPS (JOUR) | Q cumulé (gramme) ion acti | 100*Q/Q0 % |
|---|---|---|
| 0.12 | 0.007 | 0.25 |
| 0.29 | 0.008 | 0.27 |
| 1.00 | 0.014 | 0.49 |
| 2.00 | 0.019 | 0.64 |
| 6.00 | 0.028 | 0.94 |
| 8.00 | 0.037 | 1.25 |
| 12.00 | 0.043 | 1.47 |
| 15.00 | 0.045 | 1.51 |
| 21.00 | 0.060 | 2.02 |
| 26.00 | 0.064 | 2.15 |
| 33.00 | 0.076 | 2.56 |
| 40.00 | 0.091 | 3.06 |
| 49.00 | 0.108 | 3.65 |
| 61.00 | 0.114 | 3.84 |
| 79.00 | 0.153 | 5.14 |
| 96.00 | 0.180 | 6.08 |
| 117.00 | 0.205 | 6.92 |

TABLEAU 13

| TEMPS (JOUR) | Q cumulé (gramme) ion acti | 100*Q/Q0 % |
|---|---|---|
| 0.12 | 0.009 | 0.28 |
| 0.29 | 0.008 | 0.28 |
| 1.00 | 0.014 | 0.47 |
| 2.00 | 0.019 | 0.65 |
| 6.00 | 0.032 | 1.06 |
| 8.00 | 0.042 | 1.39 |
| 12.00 | 0.045 | 1.51 |
| 15.00 | 0.048 | 1.60 |
| 21.00 | 0.060 | 2.01 |
| 26.00 | 0.061 | 2.04 |
| 33.00 | 0.074 | 2.50 |
| 40.00 | 0.086 | 2.87 |
| 49.00 | 0.097 | 3.24 |
| 61.00 | 0.094 | 3.13 |
| 79.00 | 0.135 | 4.51 |
| 96.00 | 0.158 | 5.28 |
| 117.00 | 0.174 | 5.84 |

On immerge dans 500 ml d'eau distillée, thermostatée à 20°C, un cylindre dont les caractéristiques sont identiques à celui de l'exemple 12.

Le tableau 14 rassemble les résultats de la cinétique d'élution. La courbe Q/Qo = f(t), ainsi que le Te, sont reportés sur la figure 4 en annexe.

Exemple 15.

A 100 parties de la composition élastomère préparée conformément au mode opératoire décrit à l'exemple 1, on incorpore 40 parties de $KIO3$ de granulométrie 100-200 µm.

La préparation de l'échantillon est réalisée conformément au protocole décrit dans l'exemple 12.

On immerge dans 500 ml d'eau distillée, thermostatée à 20°C, un cylindre dont les caractéristiques sont les suivants :

* Diamètre : 23 mm

* Hauteur : 50 mm
* Surface : 44,4 $cm^2$
* Volume : 20,76 $cm^3$
* S/V : 2,14 $cm^{-1}$
* Masse totale : 27,2 g.
* Quantité initiale de I (Qo) : 4,62 g.

Le tableau 15 rassemble les résultats de la cinétique d'élution. Le Te calculé de cet essai est de 1760 jours.

Exemple 16.

A 100 parties de la composition élastomère préparée conformément au mode opératoire défini à l'exemple 1, on incorpore 25 parties de KIO3 de granulométrie 100-200 μm.

La préparation de l'échantillon est réalisée conformément au protocole décrit à l'exemple 12.

On immerge dans 200 ml d'eau distillée, thermostatée à 20°C, un cylindre dont les caractéristiques sont les suivantes :

TABLEAU 14

| TEMPS (JOUR) | Q cumulé (gramme) ion acti | 100*Q/Q0 % |
|---|---|---|
| 0.12 | 0.015 | 0.50 |
| 0.29 | 0.015 | 0.50 |
| 1.00 | 0.022 | 0.73 |
| 2.00 | 0.024 | 0.79 |
| 6.00 | 0.033 | 1.08 |
| 8.00 | 0.042 | 1.38 |
| 12.00 | 0.045 | 1.49 |
| 15.00 | 0.048 | 1.58 |
| 21.00 | 0.055 | 1.82 |
| 26.00 | 0.056 | 1.85 |
| 33.00 | 0.070 | 2.30 |
| 40.00 | 0.076 | 2.51 |
| 49.00 | 0.086 | 2.84 |
| 61.00 | 0.083 | 2.74 |
| 79.00 | 0.119 | 3.94 |
| 96.00 | 0.139 | 4.61 |
| 117.00 | 0.154 | 5.11 |

TABLEAU 15

| TEMPS (JOUR) | Q cumulé (gramme) ion acti | 100*Q/Q0 % |
|---|---|---|
| 0.25 | 0.031 | 0.66 |
| 0.98 | 0.043 | 0.93 |
| 2.91 | 0.055 | 1.18 |
| 4.00 | 0.059 | 1.28 |
| 8.26 | 0.075 | 1.63 |
| 9.97 | 0.076 | 1.64 |
| 13.96 | 0.087 | 1.87 |
| 17.24 | 0.086 | 1.87 |
| 20.96 | 0.079 | 1.71 |
| 22.97 | 0.082 | 1.76 |
| 27.94 | 0.095 | 2.06 |
| 39.15 | 0.104 | 2.25 |
| 45.25 | 0.118 | 2.55 |
| 53.22 | 0.102 | 2.20 |
| 65.97 | 0.141 | 3.04 |

* Diamètre : 10 mm
* Hauteur : 20 mm
* Surface : 8,3 $cm^2$
* Volume : 1,72 $cm^3$
* S/V 4,8 $cm^{-1}$
* Masse totale : 2,04 g
* Quantité initiale de I (Qo) : 0,242 g

Le tableau 16 rassemble les résultats de la cinétique d'élution. Le Te calculé de cet essai est de 530 jours.

Exemple 17.

On immerge dans 600 l d'eau distillée, thermostatée à 20°C, circulant avec un débit de 600 l/h, 1020 cylindres de même composition que celle décrite à l'exemple 16.

La quantité initiale de I (Qo) est égale à 246,8 g.

On observe bien un flux journalier correspondant à la somme des flux des cylindres élémentaires.

Le tableau 17 rassemble les résultats de la cinétique d'élution. Le Te de cet essai est de 530 jours.

| TEMPS (JOUR) | Q CUMULE (Gramme) ION ACTIF | 100*Q/Qo % |
|---|---|---|
| 1 | 4,5 | 1.8 |
| 2 | 7,65 | 3.08 |
| 7 | 12,25 | 4.93 |
| 14 | 16 | 6.44 |
| 24 | 20,25 | 8.15 |
| 30 | 22,5 | 9.06 |
| 40 | 26,25 | 10.57 |
| 50 | 30,12 | 12.13 |
| 60 | 35,0 | 14.1 |
| 70 | 38,75 | 15.6 |
| 80 | 42,5 | 17.1 |
| 90 | 45 | 18.1 |

TABLEAU 17

TABLEAU 16

| TEMPS (JOUR) | Q cumulé (gramme) ion acti | 100*Q/Q0 % |
| --- | --- | --- |
| 0.71 | 0.003 | 1.25 |
| 1.71 | 0.004 | 1.72 |
| 4.75 | 0.006 | 2.61 |
| 6.74 | 0.007 | 2.86 |
| 8.71 | 0.008 | 3.37 |
| 11.90 | 0.009 | 3.58 |
| 18.79 | 0.012 | 5.13 |
| 20.72 | 0.012 | 5.05 |
| 33.00 | 0.012 | 4.99 |
| 42.97 | 0.016 | 6.72 |
| 56.00 | 0.021 | 8.97 |
| 77.77 | 0.021 | 8.74 |

## Revendications

1. Composition de silicone polyaddition durcissable en un élastomère par des réactions d'hydrosilylation, caractérisée en ce qu'elle comporte :

— (A) : au moins un organopolysiloxane présentant par molécule au moins deux groupes vinyle liés au silicium, et ayant une viscosité inférieure à 500000 mPa · s,

— (B) : au moins un organopolysiloxane présentant par molécule au moins trois atomes d'hydrogène liés au silicium, le rapport molaire des atomes d'hydrogène liés au silicium dans (B) aux radicaux vinyle liés au silicium dans (A) étant compris entre 0,4 et 10,

— (C) : une quantité catalytiquement efficace d'un catalyseur qui est un composé d'un métal du groupe du platine,

— (D) : un composé de l'iode organique et/ou minéral à l'exception de l'iode moléculaire $I_2$, sous forme solide à température ambiante, soluble dans l'eau, non toxique, et n'inhibant pas le durcissement de ladite composition, dans laquelle ce composé de l'iode est dispersé de façon homogène.

2. Composition selon la revendication 1, caractérisée en ce qu'elle comporte de 5 à 130 parties en poids de composé d'iode (D) pour 100 parties de la somme de (A) + (B).

3. Composition de silicone selon la revendication 1 ou 2, caractérisée en ce que le composé de l'iode (D) est un iodure ou iodate de formules générales :

$$(M^{a+}) (I^-)_a \text{ et } (M^{a+}) (IO_3^-)_a$$

dans lesquelles a est un nombre entier supérieur ou égal à 1 et M est un cation choisi parmi un métal alcalin, un métal alcalino-terreux, un métal de transition et un ammonium quaternaire $(NT_4)^+$ dont les radicaux T identiques ou différents représentent un radical alkyle linéaire ou ramifié en $C_1$-$C_{20}$ ou un atome d'hydrogène.

4. Composition selon la revendication 1, caractérisée en ce que le composé de l'iode (D) est choisi parmi:

| | |
|---|---|
| $Na\,I$, | $Na\,IO_3$, |
| $K\,I$, | $K\,IO_3$, |
| $Mg\,I_2$, | $Mg\,I_2 \cdot 8H_2O$, |
| $Mg(IO_3)_2 \cdot 4H_2O$, | |
| $NH_4\,I$ | |
| $Fe\,I_2 \cdot 4H_2O$ | |
| $Mn\,I_2$ | |

5. Composition selon l'une quelconque des revendications précédentes, caractérisée en ce qu'elle comporte :

A) au moins un organopolysiloxane présentant des motifs siloxyles de formule :

$$Y_a\ Z_b\ SiO_{\frac{4-a-b}{2}} \qquad (1)$$

dans laquelle :

Y est un groupe vinyle,

Z est un groupe hydrocarboné monovalent n'ayant pas d'action défavorable sur l'activité du catalyseur, a est 1 ou 2 ; b est 0 1 ou 2 et a + b est compris entre 1 et 3, éventuellement, tous les autres motifs étant des motifs de formule moyenne :

$$Z_cSiO_{\frac{4-c}{2}} \qquad (2)$$

dans laquelle Z a la même signification que ci-dessus et c a une valeur comprise entre 0 et 3.

B) au moins un organohydrogénopolysiloxane comportant des motifs siloxyles de formule :

$$H_dW_eSiO_{\frac{4-d-e}{2}} \qquad (3)$$

dans laquelle W répond à la même définition que ci-dessus pour Z, d est 1 ou 2, c est 0, 1 ou 2, d + e a une valeur comprise entre 1 et 3, éventuellement tous les autres motifs étant des motifs de formule moyenne :

$$W_g\ SiO_{\frac{4-g}{2}}$$

dans laquelle W a la même signification que ci-dessus et g a une valeur comprise entre 0 et 3.

C) une quantité catalytiquement efficace d'une composé du platine.

D) un composé d'iode, tel que défini ci-avant.

6. Composition selon l'une quelconque des revendications précédentes, caractérisée en ce qu'elle comporte :

— (A) : 100 parties d'une huile diorganopolysiloxane bloquée à chaque extrémité de sa chaîne par un motif vinyldiorganosiloxyle dont les radicaux organiques liés aux atomes de silicium sont choisis parmi les radicaux méthyle, éthyle et phényle, au moins 60% molaire de ces radicaux étant des radicaux méthyle de viscosité 100 à 100 000 mPa · s à 25°C,

— (B) : au moins un organohydrogénopolysiloxane choisi parmi les homopolymères et les copolymères liquides linéaires ou en réseau présentant par molécule au moins 3 atomes d'hydrogène liés à des atomes de silicium différents et dont les radicaux organiques liés aux atomes de silicium sont choisis parmi les

radicaux méthyle, éthyle et 60% au moins de ces radicaux étant des radicaux méthyle, le produit (B) étant utilisé en quantité telle que le rapport molaire des fonctions hydrure sur les groupes vinyle sont compris entre 1,1 et 4,

— (C) : une quantité catalytiquement efficace d'un catalyseur au platine,

— (D) : 5 à 130 parties du composé de l'iode.

7. Composition selon la revendication 6, caractérisée en ce que 50% en poids du polymère (A) est remplacé par un copolymère en réseau comportant les motifs triméthylsiloxyle, méthylvinylsiloxyle et $SiO_{4/2}$ dans lequel 2,5 à 10% molaire des atomes de silicium comportent un groupe vinyle et dans lequel le rapport molaire des groupes triméthylsiloxane ou groupe $SiO_{4/2}$ est compris entre 0,5 et 1.

8. Composition selon l'une quelconque des revendications précédentes, caractérisée en ce qu'elle comporte en outre de 5 à 100 parties de charges siliceuses renforçantes ou semi-renforçantes pour 100 parties de la somme des organopolysiloxanes (A) + (B).

9. Composition selon l'une quelconque des revendications précédentes durcie en un élastomère.

10. Procédé de traitement d'eau, caractérisé en ce qu'on immerge une quantité adaptée d'un élastomère tel que défini à la revendication 9, en vue de libérer dans l'eau une quantité continue et contrôlée d'iode.

## Patentansprüche

1. Polyadditions-Silicon-Zusammensetzung, die durch Hydrosilylierungsreaktionen zu einem Elastomeren härtbar ist, dadurch gekennzeichnet, daß sie umfaßt

(A) zumindest ein Organopolysiloxan, das je Molekül zumindest zwei an das Silicium gebundene Vinylgruppen besitzt und eine Viskosität von niedriger als 500000 mPa · s aufweist,

(B) zumindest ein Organopolysiloxan, das je Molekül zumindest drei an das Silicium gebundene Wasserstoffatome besitzt, wobei daß Molverhältnis der an das Silicium in (B) gebundenen Wasserstoffatome zu den an das Silicium in (A) gebundenen Vinylgruppen zwischen 0,4 und 10 liegt,

(C) eine katalytischwirksame Menge eines Katalysators, der eine Verbindung eines Metalls der Platingruppe ist,

(D) eine organische und/oder mineralische Jodverbindung mit Ausnahme von molekularem Jod $J_2$ in bei Ramtemperatur fester Form, in Wasser löslich, nichttoxisch und die Härtung der Zusammensetzung, in welcher diese Jodverbindung homogen dispergiert ist, nicht inhibierend.

2. Zusammensetzung gemäß Anspruch 1, dadurch gekennzeichnet, daß sie 5 bis 130 Gew.Teile Jodverbindung (D) je 100 Teile der Summe (A) + (B) enthält.

3. Silicon-Zusammensetzung gemäß Anspruch 1 oder 2, dadurch gekennzeichnet, daß die Jodverbindung (D) ein Jodid oder Jodat der allgemeinen Formeln

$$(M^{a+})\,(J^-)_a \text{ und } (M^{a+})\,(JO_3^-)_a$$

ist, worin a eine ganze Zahl von höher oder gleich 1 ist und M ein Kation darstellt, ausgewählt unter einem Alkalimetall, einem Erdalkalimetall, einem Übergangsmetall und einem quaternären Ammonium $(NT_4)^+$, dessen Reste T, die gleich oder verschieden sind, einen linearen oder verzweigten $C_{1-20}$-Alkylrest oder ein Wasserstoffatom bedeuten.

4. Zusammensetzung gemäß Anspruch 1, dadurch gekennzeichnet, daß die Jodverbindung (D) ausgewählt ist unter

| | |
|---|---|
| Na J, | Na $JO_3$, |
| K J, | K $JO_3$, |
| Mg $J_2$, | Mg $J_2 \cdot 8H_2O$, |
| $Mg(JO_3)_2 \cdot 4H_2O$, | |
| $NH_4$ J | |
| Fe $J_2 \cdot 4H_2O$ | |
| Mn $J_2$. | |

5. Zusammensetzung gemäß einem der vorhergehenden Ansprüche, dadurch gekennzeichnet, daß sie enthält

(A) zumindest ein Organopolysiloxan mit Siloxygruppen der Formel

$$Y_a\ Z_b\ SiO_{\frac{4-a-b}{2}} \qquad (1)$$

worin
Y eine Vinylgruppe ist,
Z eine einwertige Kohlenwasserstoffgruppe ist, die keinen ungünstigen Einfluß auf die Aktivität des Katalysators ausübt,
a 1 oder 2 ist, b 0,1 oder 2 ist und a + b zwischen 1 und 3 liegen, wobei gegebenenfalls sämtliche anderen Gruppen Gruppen der durchnittlichen Formel

$$Z_c SiO_{\frac{4-c}{2}} \qquad (2)$$

sind, worin Z die gleiche Bedeutung wie vorstehend besitzt und c einen Wert zwischen 0 und 3 aufweist,
(B) zumindest ein Organohydrogenopolysiloxan mit Siloxygruppen der Formel

$$H_d W_e SiO_{\frac{4-d-e}{2}} \qquad (3)$$

worin W der vorstehend für Z angegebenen Definition entspricht, d 1 oder 2 ist, c 0,1 oder 2 Lst, d + e einen Wert zwischen 1 und 3 besitzen, wobei gegebenenfalls sämtliche anderen Gruppen Gruppen der durchschnittlichen Formel

$$W_g\ SiO_{\frac{4-g}{2}}$$

sind, in der W die gleiche Bedeutung wie vorstehend besitzt und g einen Wert zwischen 0 und 3 wiedergibt,
(C) eine katalytisch wirksame Menge einer Platinverbindung,
(D) eine Jodverbindung, wie vorstehend definiert.

6. Zusammensetzung gemäß einem der vorhergehenden Ansprüche, dadurch gekennzeichnet, daß sie enthält
(A) 100 Teile eines Diorganopolysiloxanöls, welches blockiert ist an jedem seiner Kettenenden durch eine Vinyldiorganosiloxygruppe, deren organische, an die Siliciumatome gebundenen Reste ausgewählt sind unter den Methyl-, Ethyl- und Phenylresten, wobei zumindest 60 Mol-% dieser Reste Methylreste sind, mit einer Viskosität von 100 bis 100000 mPa · s bei 25°C,
(B) zumindest ein Organohydrogenopolysiloxan, ausgewählt unter den flüssigen, linearen oder netzförmigen Homopolymeren und Copolymeren, die je Molekül zumindest 3 an verschiedene Siliciumatome gebundene Wasserstoffatome besitzen und deren organische, an die Siliciumatome gebundenen Reste ausgewählt sind unter den Methyl- und Ethylresten, wobei zumindest 60% dieser Reste Methylreste sind, und wobei das Produkt (B) in einer derartigen Menge eingesetzt wird, daß das Molverhältnis der Hydridfunktionen zu den Vinylgruppen zwischen 1,1 und 4 liegt,
(C) eine katalytisch wirksame Menge eines Platinkatalysators,
(D) 5 bis 130 Teile der Jodverbindung.

7. Zusammensetzung gemäß Anspruch 6, dadurch gekennzeichnet, daß 50 Gew.% des Polymeren (A) durch ein netzförmiges Copolymeres ersetzt sind, das die Trimethylsiloxy-, Methylvinylsiloxy- und $SiO_{4/2}$-Gruppierungen enthält, worin 2,5 bis 10 Mol-% der Siliciumatome eine Vinylgruppe aufweisen und worin das Molverhältnis der Trimethylsiloxangruppen zur Gruppe $SiO_{4/2}$ zwischen 0,5 und 1 liegt.

8. Zusammensetzung gemäß einem der vorhergehenden Ansprüche, dadurch gekennzeichnet, daß sie außerdem 5 bis 100 Teile verstärkende oder halbverstärkende, siliciumdioxidhaltige Füllstoffe je 100 Teile der Summe der Organopolysiloxane (A) + (B) enthält.

9. Zusammensetzung gemäß einem der vorhergehenden Ansprüche, zu einem Elastomeren gehärtet.

10. Verfahren zur Behandlung von Wasser, dadurch gekennzeichnet, daß man eine geeignete Menge eines Elastomeren, wie in Anspruch 9 definiert, eintaucht, um in dem Wasser eine kontinuierliche und Kontrollierte Jodmenge freizusetzen.

## Claims

1. Polyaddition silicone composition curable to form an elastomer by means of hydrosilylation reactions, characterised in that it comprises :

— (A) : at least one organopolysiloxane having, per molecule, at least two vinyl groups bonded to silicon and having a viscosity of less than 500,000 mPas,

— (B) : at least one organopolysiloxane having, per molecule, at least three hydrogen atoms bonded to silicon, the molar ratio of the hydrogen atoms bonded to silicon in (B) to the vinyl radicals bonded to silicon in (A) being between 0.4 and 10,

— (C) : a catalytically effective quantity of a catalyst which is a compound of a metal of the platinum group, and

— (D) : an organic and/or inorganic iodine compound, with the exception of molecular iodine $I_2$, which is in solid form at room temperature, soluble in water, nontoxic and does not inhibit the curing of the said composition, in which this iodine compound is dispersed homogeneously.

2. Composition according to Claim 1, characterised in that it comprises from 5 to 130 parts by weight of iodine compound (D) per 100 parts by weight of the sum of (A) + (B).

3. Silicone composition according to Claim 1 or 2, characterised in that the iodine compound (D) is an iodide or iodate of general formulae :

$$(M^{a+})\,(I^-)_a \text{ and } (M^{a+})\,(IO_3^-)_a$$

in which formulae a is an integer greater than or equal to 1 and M is a cation chosen from an alkali metal, an alkaline-earth metal, a transition metal and a quaternary ammonium $(NT_4)^+$, in which the radicals T, which may be identical or different, represent a $C_1$-$C_{20}$, straight-chain or branched alkyl radical or a hydrogen atom.

4. Composition according to Claim 1, characterised in that the iodine compound (D) is chosen from :

| | |
|---|---|
| NaI, | $NaIO_3$, |
| KI, | $KIO_3$, |
| $MgI_2$, | $MgI_2 \cdot 8H_2O$, |
| $Mg(IO_3)_2 \cdot 4H_2O$, | |
| $NH_4I$, | |
| $FeI_2 \cdot 4H_2O$ and | |
| $MnI_2$ | |

5. Composition according to any one of the preceding claims, characterised in that it comprises :

A) at least one organopolysiloxane having siloxy units of the formula :

$$Y_aZ_bSiO_{\frac{4-a-b}{2}} \qquad (1)$$

in which :

Y is a vinyl group,

Z is a monovalent hydrocarbon group not having an adverse action on the activity of the catalyst, a is 1 or 2 ; b is 0,1 or 2 and a + b is between 1 and 3, all of the other units optionally being units of average formula:

$$Z_cSiO_{\frac{4-c}{2}} \qquad (2)$$

in which Z has the same meaning as above and c has a value of between 0 and 3,

B) at least one organohydropolysiloxane comprising siloxy units of formula :

$$H_dW_eSiO_{\frac{4-d-e}{2}} \qquad (3)$$

in which W corresponds to the same definition as above for Z, d is 1 or 2, c is 0,1 or 2 and d + e has a value of between 1 and 3, all of the other units optionally being units of average formula :

$$W_gSiO_{\frac{4-g}{2}}$$

in which W has the same meaning as above and g has a value of between 0 and 3,
C) a catalytically effective quantity of a platinum compound, and
D) an iodine compound as defined above.

6. Composition according to any one of the preceding claims, characterised in that it comprises :
— (A) : 100 parts of a diorganopolysiloxane oil blocked at each end of its chain by a vinyldiorganosiloxy unit in which the organic radicals bonded to the silicon atoms are chosen from methyl, ethyl and phenyl radicals, at least 60 mol% of these radicals being methyl radicals, having a viscosity of 100 to 100,000 mPas at 25°C,
— (B) : at least one organohydropolysiloxane chosen from linear or crosslinked liquid homopolymers and copolymers having per molecule at least 3 hydrogen atoms bonded to different silicon atoms and in which the organic radicals bonded to silicon atoms are chosen from methyl and ethyl radicals and at least 60% of these radicals being methyl radicals, the product (B) being used in a quantity such that the molar ratio of the hydride functional groups to the vinyl groups is between 1.1 and 4,
— (C) : a catalytically effective quantity of a platinum catalyst, and
— (D) : 5 to 130 parts of the iodine compound.

7. Composition according to Claim 6, characterised in that 50% by weight of the polymer (A) is replaced by a crosslinked copolymer comprising trimethylsiloxy, methylvinylsiloxy and $SiO_{4/2}$ units in which 2.5 to 10 mol% of the silicon atoms carry a vinylgroup and in which the molar ratio of the trimethylsiloxane groups to the $SiO_{4/2}$ group is between 0.5 and 1.

8. Composition according to any one of the preceding claims, characterised in that it also comprises from 5 to 100 parts of reinforcing or semireinforcing siliceous fillers per 100 parts of the sum of the organopolysiloxanes (A) + (B).

9. Composition according to any one of the preceding claims cured to form an elastomer.

10. Process for water treatment, characterised in that a suitable quantity of an elastomer as defined in Claim 9 is immersed with a view to releasing a continuous and controlled amount of iodine into the water.

FIGURE 1

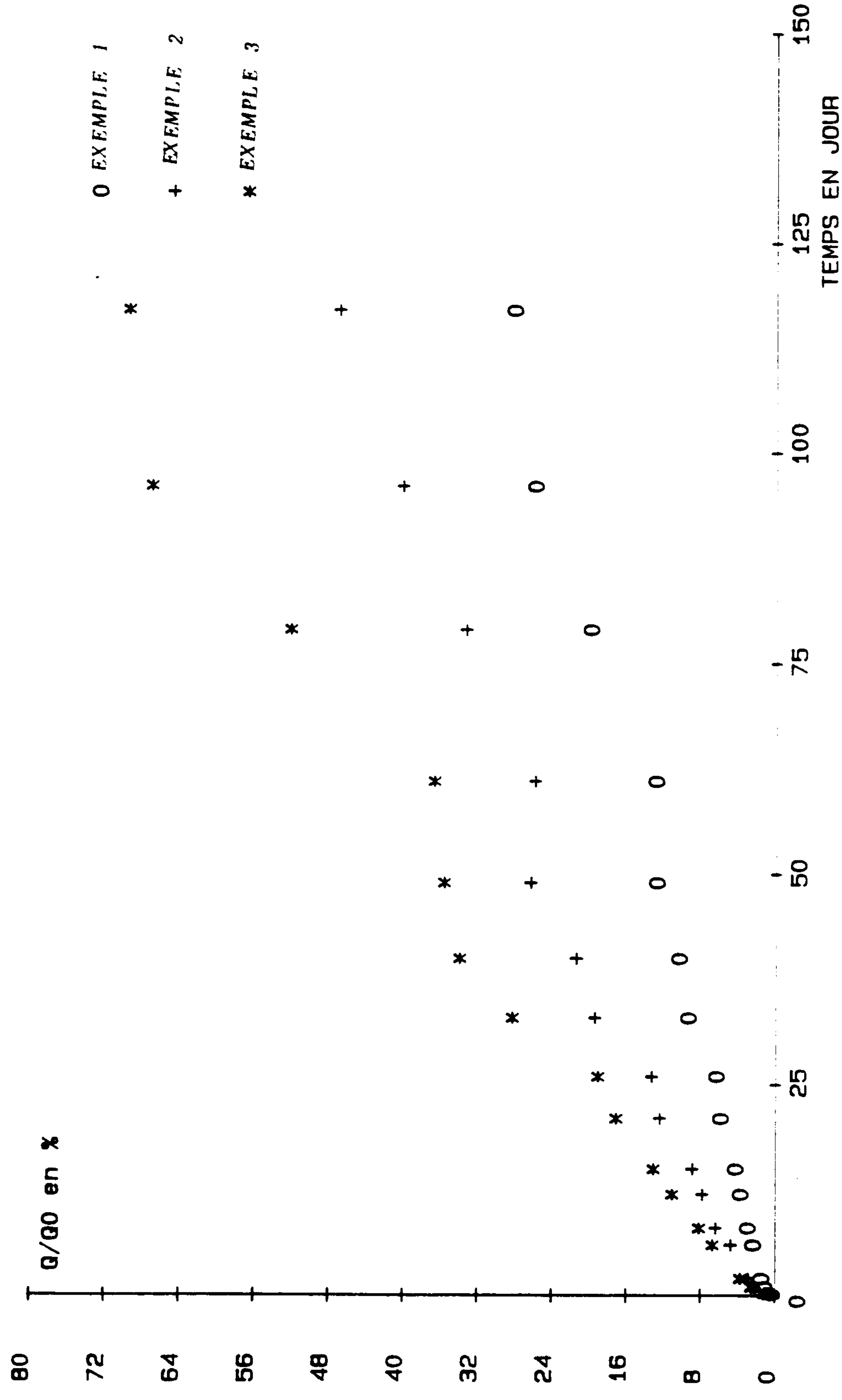
Q/Q0 en %
80
72
64
56
48
40
32
24
16
8
0
0
25
50
75
100
125
150
TEMPS EN JOUR
0 EXEMPLE 1
+ EXEMPLE 2
* EXEMPLE 3

FIGURE 2

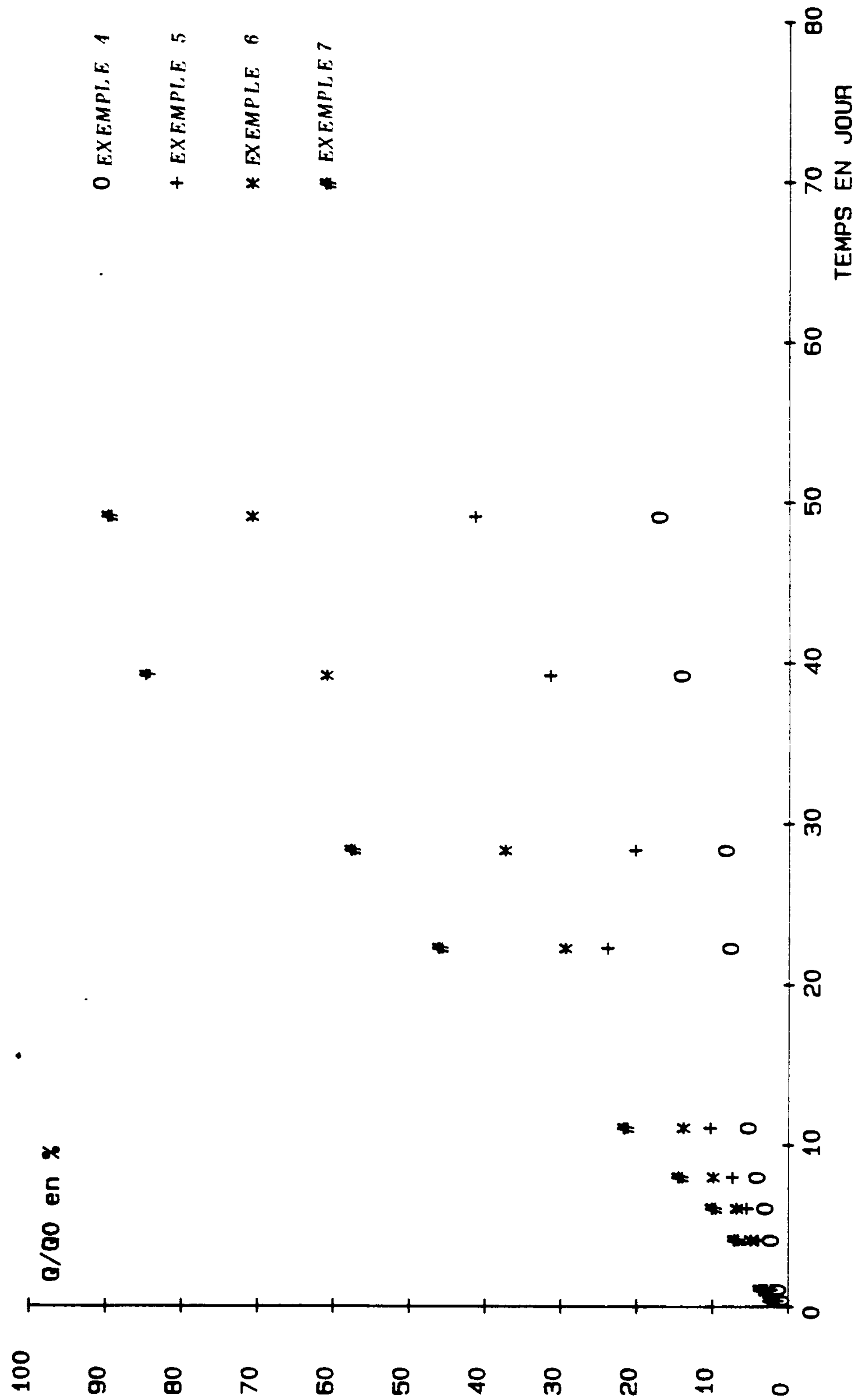
Q/Q0 en %
TEMPS EN JOUR
0 EXEMPLE 4
+ EXEMPLE 5
* EXEMPLE 6
# EXEMPLE 7
0
10
20
30
40
50
60
70
80
90
100
0
10
20
30
40
50
60
70
80

FIGURE 3

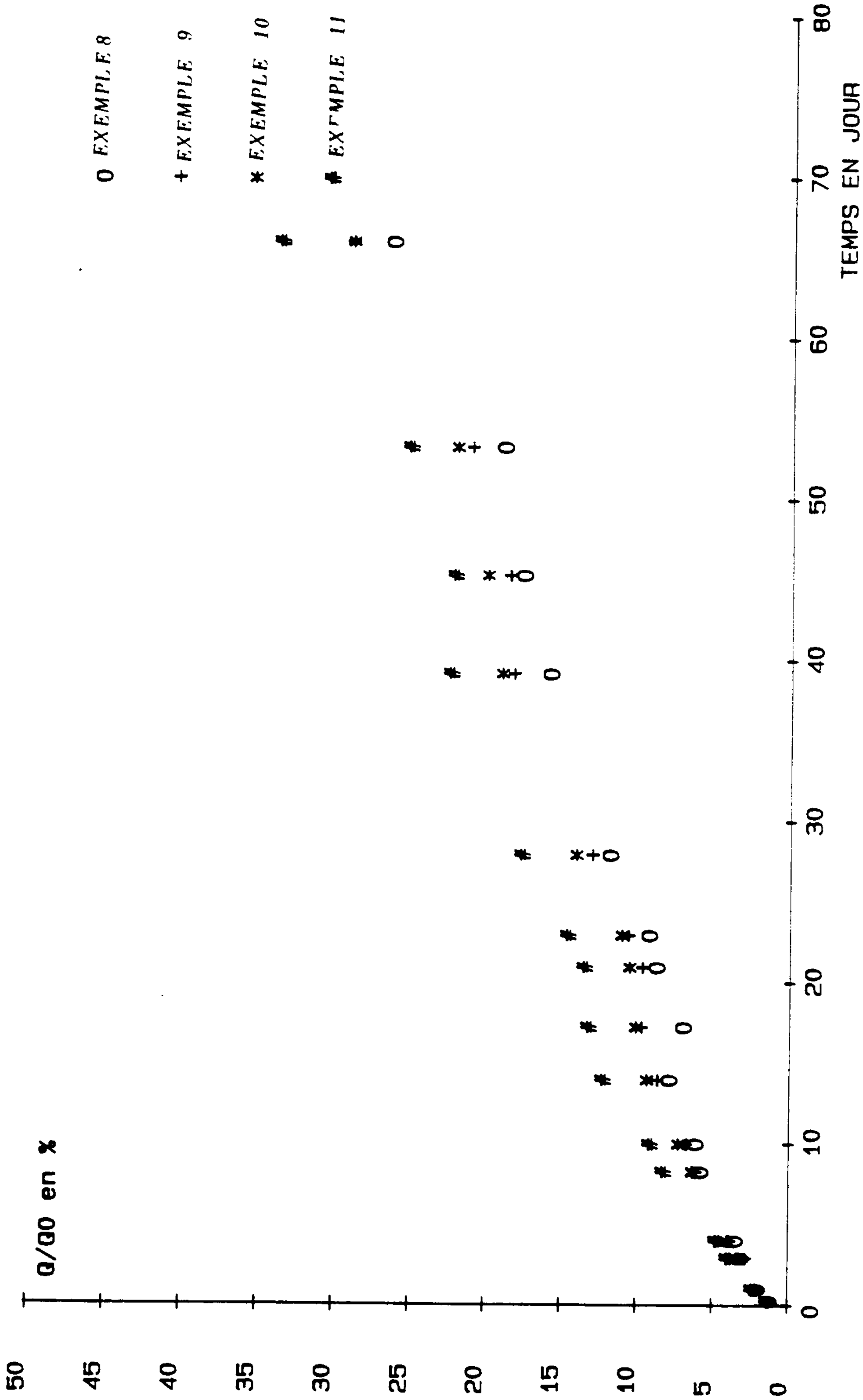
Q/Q0 en %
50
45
40
35
30
25
20
15
10
5
0
0
10
20
30
40
50
60
70
80
TEMPS EN JOUR
O EXEMPLE 8
+ EXEMPLE 9
* EXEMPLE 10
# EXEMPLE 11

FIGURE 4

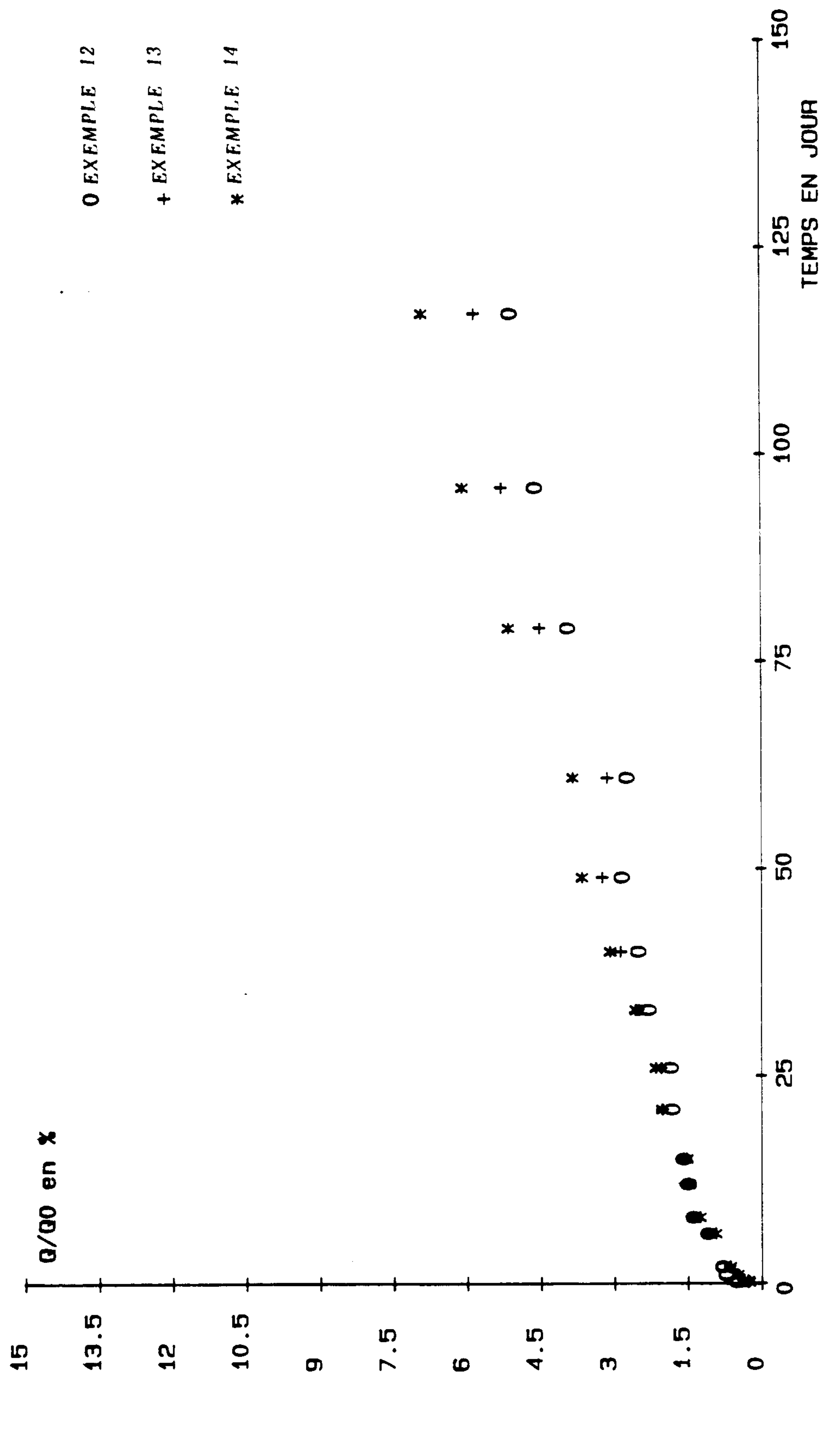
Q/Q0 en %
15
13.5
12
10.5
9
7.5
6
4.5
3
1.5
0
0
25
50
75
100
125
150
TEMPS EN JOUR
O EXEMPLE 12
+ EXEMPLE 13
* EXEMPLE 14